# EUROPÄISCHE PATENTSCHRIFT

(11) **EP 3 209 633 B1**
(45) Veröffentlichungstag und Bekanntmachung des Hinweises auf die Patenterteilung: **28.11.2018**
(21) Anmeldenummer: 16745764.7
(22) Anmeldetag: 03.08.2016
(51) Int. Cl.: C07C 67/10, C07C 67/54, C07C 69/24, C07C 69/01, C07C 51/09, C07C 53/08

(54) **VERFAHREN ZUR KATALYTISCHEN UMVINYLIERUNG VON CARBONSÄUREN**
PROCESS FOR CATALYTIC TRANSVINYLATION OF CARBOXYLIC ACIDS
PROCÉDÉ DE TRANSVINYLATION CATALYSÉE D'ACIDES CARBOXYLIQUES

(30) Priorität: 27.08.2015 DE 102015216373
(43) Veröffentlichungstag der Anmeldung: 30.08.2017
(73) Patentinhaber: Wacker Chemie AG, 81737 München (DE)
(72) Erfinder: SCHULTHEISS, Peter, 84489 Burghausen (DE); HOELLEIN, Volker, 84489 Burghausen (DE); PATSCH, Brigitte, 84489 Burghausen (DE)
(74) Vertreter: Schuderer, Michael
(86) Internationale Anmeldenummer: PCT/EP2016/068521
(87) Internationale Veröffentlichungsnummer: WO 2017/032572

(56) Entgegenhaltungen:
- WO-A1-2013/117295
- WO-A1-2015/078746
- WO-A1-2015/078747

## Beschreibung

Die Erfindung betrifft ein Verfahren zur Umvinylierung einer Eduktcarbonsäure mit einem Eduktvinylester zu einem Produktvinylester und der korrespondierenden Säure des Eduktvinylesters in Gegenwart von einem oder mehreren Katalysatoren, wobei der Eduktvinylester, die Eduktcarbonsäure und der Katalysator einem Reaktor zugeführt werden, und die Umvinylierungsreaktion durchgeführt wird, und nach Abschluss der Umvinylierungsreaktion der Produktvinylester destillativ abgetrennt wird.

Die Umvinylierung von Carbonsäuren dient der Darstellung von Vinylestern. Darunter versteht man die Übertragung einer Vinyl-Einheit eines Eduktvinylesters (1V) auf eine Eduktcarbonsäure (2S) unter Generierung eines Produktvinylesters (2V) und der korrespondierenden Säure des Eduktvinylesters (1S).

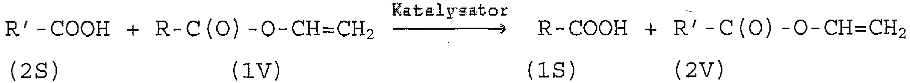

In der WO 92/09554 A1 wird ein Verfahren zur katalytischen Umvinylierung beschrieben, bei dem nach der Umvinylierung aus dem Reaktionsgemisch in einem ersten Schritt Eduktcarbonsäure und Katalysator abgetrennt werden, und der Produktvinylester anschließend nach Zugabe von Wasser mittels Azeotropdestillation abgetrennt wird.

Die US 5,210,207 beschreibt ein Umvinylierungsverfahren bei welchem zur Beschleunigung der Reaktion und zur Erhöhung deren Selektivität mittels Reaktivdestillation mindestens einer der Produktkomponenten aus dem Reaktionsgemisch entfernt wird.

Die WO 2011/139360 A1 beschreibt ein Verfahren zur kontinuierlichen, katalytischen Umvinylierung von einer Carbonsäure mit Vinylacetat zu einem Produktvinylester und der korrespondierenden Essigsäure in einer Reaktivdestillationskolonne, wobei zur Verschiebung des Reaktionsgleichgewichts kontinuierlich ein Gemisch aus Vinylacetat und Essigsäure aus der Reaktionszone entnommen wird. Der Produktvinylester wird am Kolonnenboden als Gemisch entnommen, welches noch Katalysator sowie Eduktcarbonsäure und Vinylacetat enthält. Dieses Gemisch wird in einem Kondensator verflüssigt und der Produktvinylester als Sumpfprodukt entnommen.

Die WO 2011/139361 A1 beschreibt ein Verfahren zur semikontinuierlichen, katalytischen Umvinylierung von einer Carbonsäure mit Vinylacetat zu einem Produktvinylester und der korrespondierenden Essigsäure in einem Rührreaktor, wobei zur Verschiebung des Reaktionsgleichgewichts kontinuierlich ein Gemisch aus Vinylacetat und Essigsäure aus der Reaktionszone entnommen wird. Der Produktvinylester wird am Boden des Rührreaktors als Gemisch entnommen, welches noch Katalysator sowie Eduktcarbonsäure und Vinylacetat enthält. Dieses Gemisch wird mittels fraktionierter Destillation aufgetrennt, wobei zunächst Vinylacetat abdestilliert wird und nach Druck/Temperatur-Erhöhung der Produktvinylester abdestilliert wird.

Die WO 2013/117294 A1 beschreibt ein kontinuierliches, katalytisches Verfahren zur Umvinylierung einer Carbonsäure mit einem Eduktvinylester zu einem Produktvinylester und der korrespondierenden Säure. Das Reaktionsgemisch wird einem Entspannungsgefäß zugeführt und dort auf Normaldruck entspannt. Das Reaktionsgemisch wird vor der Entspannung gekühlt um möglichst alle Komponenten bei der Entspannung flüssig zu halten. Eine gegebenenfalls gebildete Gasphase wird in die zweite Trennvorrichtung geleitet. Anschließend wird das flüssige Reaktionsgemisch in einer ersten Trennvorrichtung aufgetrennt, wobei am Kopf ein Gemisch aus Eduktvinylester, Produktvinylester und korrespondierender Säure entnommen wird und einer zweiten Trennvorrichtung aufgegeben wird. In der zweiten Trennvorrichtung wird ein Gemisch aus Produktvinylester und korrespondierender Säure am Sumpf entnommen und einer dritten Trennvorrichtung zugeführt. In dieser fällt der Produktvinylester als Sumpfprodukt an.

Die WO 2013/117295 A1 beschreibt ein kontinuierliches, katalytisches Verfahren zur Umvinylierung einer Carbonsäure mit einem Eduktvinylester zu einem Produktvinylester und der korrespondierenden Säure, wobei die korrespondierende Säure in einem letzten Schritt derivatisiert wird. Das Reaktionsgemisch wird einem Entspannungsgefäß zugeführt und dort auf Normaldruck entspannt, wobei mittels Kühlung die Bildung einer Gasphase unterdrückt wird. Anschließend wird das flüssige Reaktionsgemisch in einer ersten Trennvorrichtung aufgetrennt, wobei am Kopf ein Gemisch aus Eduktvinylester, Produktvinylester und korrespondierender Säure entnommen wird und einer zweiten Trennvorrichtung aufgegeben wird. Wird bei der Entspannung eine Gasphase gebildet, wird diese auch der zweiten Trennvorrichtung aufgegeben. In der zweiten Trennvorrichtung wird ein Gemisch aus Produktvinylester und korrespondierender Säure am Sumpf entnommen und einer dritten Trennvorrichtung zugeführt. In dieser fällt der Produktvinylester als Sumpfprodukt an. Die am Kopf erhaltene Säure wird in einem nachgeschalteten Verfahren derivatisiert, beispielsweise kann Essigsäure mit Ethylen und Sauerstoff zu Vinylacetat umgesetzt werden.

In der WO 2015/078746 A1 und der WO 2015/078747 A1 wird jeweils ein Verfahren beschrieben zur Ruthenium-katalysierten Umvinylierung einer Carbonsäure mit einem Eduktvinylester zu einem Produktvinylester und der korrespondierenden Säure, wobei nach Abschluss der Umvinylierungsreaktion der Eduktvinylester und die korrespondierende Säure aus dem Reaktionsgemisch mittels Destillation abgetrennt werden, anschließend der Produktvinylester destillativ abgetrennt wird und das verbleibende Reaktionsgemisch in den Reaktor zurückgeführt wird.
Bei der Umvinylierung verbleiben aufgrund der unvollständigen Reaktion Eduktvinylester und Eduktsäure in dem Reaktionsgemisch, und es wird neben dem Zielprodukt Produktvinylester noch die korrespondierende Säure aus dem Eduktvinylester freigesetzt. Daher wird bei den Verfahren aus dem Stand der Technik das bei der Umvinylierung entstehende Reaktionsgemisch in meist mehrstufigen Destillationsverfahren in dessen Bestandteile aufgeteilt, und der Produktvinylester isoliert. Neben dem hohen apparativem Aufwand sind diese Trennverfahren zeitintensiv und erfordern einen hohen Energieaufwand.
Es bestand daher die Aufgabe, ein Verfahren zur katalytischen Umvinylierung von Carbonsäuren zur Verfügung zu stellen, mit welchem der Produktvinylester mit geringerem Aufwand, aber trotzdem mit hohem Reinheitsgrad zugänglich wird.
Gegenstand der Erfindung ist ein Verfahren zur Umvinylierung einer Eduktcarbonsäure mit einem Eduktvinylester zu einem Produktvinylester und der korrespondierenden Säure des Eduktvinylesters in Gegenwart von einem oder mehreren Katalysatoren, in einem Reaktor bei einer Temperatur von 100°C bis 170°C und einem Druck von 2 bar abs. bis 15 bar abs., wobei der Eduktvinylester, die Eduktcarbonsäure und der Katalysator einem Reaktor zugeführt werden, und die Umvinylierungsreaktion durchgeführt wird, und nach Abschluss der Umvinylierungsreaktion der Produktvinylester vom Reaktionsgemisch destillativ abgetrennt wird, dadurch gekennzeichnet, dass das Reaktionsgemisch in einem Entspannungsbehälter entspannt wird, die dabei anfallende gasförmige Phase anschließend kondensiert wird und ohne weitere Aufreinigung in den Reaktor zurückgeführt wird, und die dabei anfallende flüssige Phase in einer ersten Destillation von Eduktvinylester und korrespondierender Säure befreit wird, und in einem weiteren Destillationsschritt der Produktvinylester von dem Reaktionsgemisch abgetrennt wird.

Als Eduktvinylester können beliebige Carbonsäurevinylester der allgemeinen Formel R-C(O)-O-CH=CH₂ eingesetzt werden, wobei R ein aliphatischen Rest mit 1 bis 12 C-Atomen sein kann, oder ein cycloaliphatischer Rest mit bis zu 12 C-Atomen sein kann, oder ein aromatischer Rest mit bis zu 12 C-Atomen sein kann. Bevorzugt ist die Verwendung niedermolekularer Eduktvinylester, wobei R ein Alkylrest mit 1 bis 6 C-Atomen ist. Besonders bevorzugt ist die Verwendung von Vinylacetat.

Ferner wird dem Reaktor mindestens eine Eduktcarbonsäure der allgemeinen Formel R'-COOH zugeführt, wobei R' ein aliphatischer Rest mit 1 bis 22 C-Atomen sein kann, oder ein cycloaliphatischer Rest mit bis zu 22 C-Atomen sein kann, oder ein aromatischer Rest mit bis zu 22 C-Atomen sein kann. Bevorzugt werden Eduktcarbonsäuren der genannten Verbindungsklassen mit 6 bis 18 C-Atomen eingesetzt. Beispiele hierfür sind Capronsäure, Cyclohexancarbonsäure, n-Heptansäure, 2-Methylhexansäure, 2-Ethylhexansäure, n-Octansäure, n-Nonansäure, Isononansäure, Neononansäure, n-Decansäure, Neodecansäure, Laurinsäure, Myristinsäure, Palmitinsäure, Stearinsäure, Benzoesäure, Naphtalincarbonsäure. Besonders bevorzugt werden Versaticsäuren^{R} (alphaverzweigte Carbonsäuren mit 9 bis 12 C-Atomen der Fa. Momentive) oder Neo-Säuren mit 9 bis 12 C-Atomen und Fettsäuren wie Laurinsäure, Myristinsäure, Palmitinsäure, Stearinsäure.
Als Katalysator bevorzugt werden Ru-Verbindungen oder Pd-Verbindungen, welche typischerweise für Umvinylierungsreaktionen eingesetzt werden. Geeignete Katalysatoren sind dem Fachmann bekannt, beispielsweise aus der WO 2011/139360 A1, der WO 2011/139361 A1 und dem US-Patent 4,981,973. Bevorzugt werden Ru-Verbindungen eingesetzt. Besonders bevorzugt ist die Verwendung von Ru-Acetat oder einer aktiven Ru-Katalysatorlösung, wie sie dem Fachmann aus den Offenlegungsschriften DE 102014206915 und DE 102014206916 bekannt sind.
Der Edelmetall-Katalysator wird typischerweise in Konzentrationen von 0,1 bis 10000 ppm (Gehalt Edelmetall bezogen auf die Reaktionsmasse aus Eduktvinylester und Eduktcarbonsäure) eingesetzt, bevorzugt ist die Verwendung von 1 bis 1000 ppm (Gehalt Edelmetall bezogen auf die Reaktionsmasse aus Eduktvinylester und Eduktcarbonsäure).
Den Edukten kann gegebenenfalls ein Polymerisationsinhibitor zugesetzt werden. Bevorzugt werden 100 bis 10000 ppm, bezogen auf die Reaktionsmasse aus Eduktvinylester und Eduktcarbonsäure, Polymerisationsinhibitor eingesetzt. Beispiele für Polymerisationsinhibitoren sind Hydrochinon, Methoxyhydrochinon, tertiär-Butylcatechol, Phenothiazin (PIZ) oder Nitroxidradikale wie TEMPO oder 4-OH-TEMPO (TEMPO = 2,2,6,6-Tetramethylpiperidinyloxyl). Bevorzugt ist die Verwendung von Phenothiazin oder Hydrochinon.

Zur Umvinylierung können die Reaktanden Eduktvinylester, Eduktcarbonsäure und Katalysator, sowie gegebenenfalls Inhibitor dem Reaktor einzeln oder in einem Gemisch zugeführt werden.

Das molare Verhältnis von Eduktvinylester zu Eduktcarbonsäure kann 1 : 10 bis 10 : 1 betragen. Bevorzugt ist ein Verhältnis von Eduktvinylester zu Eduktcarbonsäure von 2 : 1 bis 8 : 1, besonders bevorzugt ist ein Verhältnis von 3 : 1 bis 6 : 1.

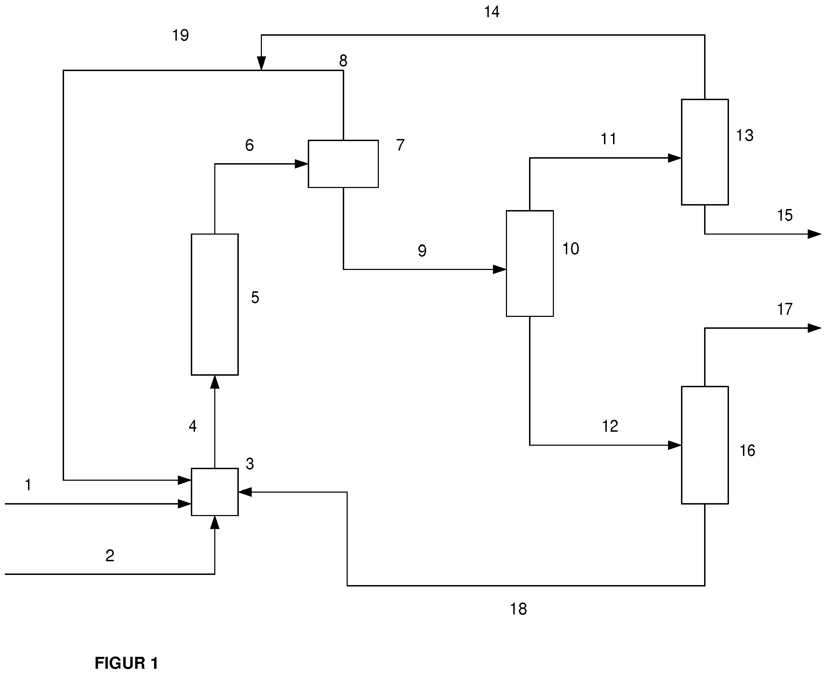

Der prinzipielle Ablauf des erfindungsgemäßen Verfahrens ist in der Figur 1 dargestellt:

Die Eduktströme von Eduktvinylester (1) und Eduktcarbonsäure (2), sowie die Rückführungsströme (18 und 19) werden gegebenenfalls einem vorgeschalteten Mischer (3) und dann dem Reaktor (5) zugeführt (4), oder direkt dem Reaktor (5) zugeführt, in welchem die Umvinylierungsreaktion stattfindet.

Die Umvinylierung wird im Allgemeinen bei einer Temperatur von 100°C bis 170°C, vorzugsweise bei einer Temperatur von 120°C bis 150°C durchgeführt. Der Druck, bei welchem die Umvinylierung erfolgt, ist von der Temperatur abhängig und beträgt im Allgemeinen 2 bar abs. bis 15 bar abs., vorzugsweise 5 bar abs. bis 15 bar abs., und am meisten bevorzugt 5 bar abs. bis 10 bar abs.. Die Reaktion wird bevorzugt in einer Schutzgasatmosphäre, beispielsweise Stickstoff, in an sich bekannter Weise, durchgeführt. Die Umvinylierung wird ohne Reaktivdestillation durchgeführt.

Als Reaktor (5) können Rührkessel, Rührkessel-Kaskaden oder Rohrreaktoren eingesetzt werden. Vorzugsweise ist der Reaktor (5) ein Rohrreaktor. Dem Reaktor (5) kann gegebenenfalls ein Mischer (3) vorgeschaltet sein, in welchem die Edukte vorgemischt werden können. Geeignete Mischer sind statische Mischer oder dynamische Mischer, wobei statische Mischer bevorzugt werden.

Die Verweilzeit im Reaktor beträgt bei dem erfindungsgemäßen Verfahren im Allgemeinen 0,25 bis 5 Stunden, vorzugsweise 1 Stunde bis 4 Stunden.

Das bei der Umvinylierung erhaltene Reaktionsgemisch (6) wird kontinuierlich oder batchweise aus dem Reaktor entnommen und in ein Entspannungsgefäß (7) entspannt. Im Allgemeinen wird auf einen Druck von 0,5 bar abs. bis 1,5 bar abs. entspannt. Vorzugsweise wird auf Normaldruck (1 bar abs.) entspannt. Geeignete Entspannungsgefäße sind dem Fachmann bekannt und im Handel beispielsweise unter der Bezeichnung Flash-Box erhältlich.

Bei der Entspannung wird eine gasförmige Phase (Dampfphase) gebildet, welche überwiegend nicht umgesetzten Eduktvinylester und noch korrespondierende Säure enthält, und es wird eine flüssige Phase gebildet, welche überwiegend Produktvinylester enthält sowie geringe Anteile an Eduktvinylester und korrespondierende Säure sowie Katalysator und polymere Nebenprodukte. Im Allgemeinen liegen nach der Entspannung 60 Gew.-% bis 90 Gew.-% des Reaktionsgemisches als Flüssigkeit vor und 10 Gew.-% bis 40 Gew.-% als gasförmige Phase vor, jeweils bezogen auf das Gesamtgewicht des entnommenen Reaktionsgemisches.

Der nach der Entspannung gasförmig vorliegende Anteil (8) wird aus dem Entspannungsgefäß entfernt, anschließend mittels Wärmetauscher gekühlt und zur Kondensation gebracht und ohne weitere Aufreinigung nach dessen Kondensation, gegebenenfalls über den vorgeschalteten Mischer (3), in den Reaktor (5) zurückgeführt.

Die nach der Entspannung flüssig vorliegende Phase (9) wird mittels Destillation aufgetrennt. Druck und Temperatur der Destillation sowie die Auslegung der Destillationskolonnen hängen von den im Produktgemisch vorliegenden Komponenten ab und können beispielsweise mittels Routineversuchen vom Fachmann ermittelt werden. Es wird keine Azeotropdestillation ausgeführt.

Von der nach der Entspannung resultierenden flüssigen Phase (9) wird in einer Destillationsvorrichtung (10) der in dieser Phase noch enthaltene Eduktvinylester und dessen korrespondierende Säure abgetrennt (11). Das dabei erhaltene Gemisch wird in einer zweiten Destillationsvorrichtung (13) in die Komponenten Eduktvinylester (14) und korrespondierende Säure (15) aufgetrennt. Anschließend kann der zurückgewonnene Eduktvinylester (14), gegebenenfalls zusammen mit der zur Kondensation gebrachten Dampfphase aus der Entspannung (8), gegebenenfalls in den vorgeschalteten Mischer oder direkt in den Reaktor (5) zurückgeführt werden (19).

Das Sumpfprodukt (12) aus der Destillationsvorrichtung (10), welches Produktvinylester, Eduktcarbonsäure, polymere Nebenprodukte, gegebenenfalls Anhydride und Katalysator enthält, wird einer weiteren Destillationsvorrichtung (16) zugeführt. Falls der Siedepunkt des Produktvinylesters niedriger ist als der der Eduktcarbonsäure wird der Produktvinylester vollständig oder teilweise als Kopfprodukt (17) von den restlichen Bestandteilen abgetrennt. Der zurückbleibende katalysatorhaltige Rest (18) wird dann in den Reaktor (5) oder gegebenenfalls über den vorgeschalteten Mischer (3) zurückgeführt.

Falls der Siedepunkt des Produktvinylesters höher ist als der der Eduktcarbonsäure wird in der Destillationsvorrichtung (16) ein Gemisch von Produktvinylester und Eduktcarbonsäure als Kopfprodukt (17) vom katalysatorhaltigen Rest abgetrennt. Dieses Gemisch wird dann in einer weiteren Destillationsvorrichtung zur Herstellung des reinen Produktvinylesters aufgetrennt.

Im Unterschied zu den Verfahren aus dem Stand der Technik wird in dem vorliegenden Verfahren nicht das gesamte, bei der Umvinylierung resultierende Reaktionsgemisch destillativ getrennt, sondern nur der Teil, welcher nach dem Entspannungsschritt in flüssiger Phase anfällt. Die Dampfphase, im Allgemeinen 10 bis 40 Gew.-% des entnommenen Reaktionsgemisches, wird ohne weitere Aufarbeitung in den Reaktor zurückgeführt.

Mit dieser Maßnahme wird der Aufwand für die Destillation deutlich reduziert, das heißt die Investitionskosten und Betriebskosten werden deutlich reduziert. Wie die nachfolgenden Beispiele zeigen werden die Ausbeuten trotz dieser Rückführung nicht negativ beeinflusst.

In den Verfahren des Stands der Technik werden die kontinuierlichen Destillationen so geführt, dass der Produktvinylester bei der Destillation als Sumpfprodukt erhalten wird. Im vorliegenden Verfahren werden alle leichter siedenden Bestandteile, im Allgemeinen der Eduktvinylester und die korrespondierende Säure, im ersten Schritt aus dem Gemisch entfernt. Falls der Siedepunkt des Produktvinylesters niedriger ist als der der Eduktcarbonsäure, was bei fast allen industriell durchgeführten Umvinylierungen der Fall ist, kann daher im darauffolgenden Destillationsschritt der Produktvinylester von der Eduktcarbonsäure und den polymeren Nebenprodukten als Kopfprodukt bei der Destillation erhalten werden und damit mit hoher Reinheit erhalten werden.

### Die nachfolgenden Beispiele dienen der weiteren Erläuterung der Erfindung:

Als Ru-Katalysator wurden jeweils 100 ppm [Ru₃O(OAc)₆(H₂O)₃]OAc in Form einer essigsauren Lösung mit 4,5 Gew.-% Ru der Firma Umicore eingesetzt.

### Vergleichsbeispiel 1:

In einem kontinuierlich betriebenen Rohrreaktor wurden 25 kg/h Vinylacetat, 6 kg/h Laurinsäure und 30 kg/h Rücklaurinsäure, welche neben Ru-Katalysator, polymeren Nebenprodukten und Vinyllaurat noch ein Gemisch aus Laurinsäure und Laurinsäureanhydrid enthielt, in den Reaktor dosiert. Das molare Verhältnis zwischen Vinylacetat und Laurinsäure betrug 4 : 1.

Die Reaktion wurde bei 140°C und 7 bar abs. durchgeführt. Es wurde kontinuierlich Reaktionsgemisch entnommen und auf Atmosphärendruck entspannt. Dabei fielen etwa 20 Gew.-% des entnommenen Reaktionsgemisches als Dampfphase an mit ca. 96 Gew.-% Vinylacetat und ca. 4 Gew.-% Essigsäure, sowie Spuren von Vinyllaurat und Laurinsäure im Gemisch. Auf die Rückführung der durch Entspannung generierten Stromes wurde verzichtet.

Bei einer Betriebszeit von ca. 1000 Stunden schwankte die Ausbeute in Abhängigkeit vom Polymer- und Katalysatorgehalt zwischen 60 % und 75 %.

Aus der nach Entspannung erhaltenen flüssigen Phase wurde in der ersten Destillation ein Gemisch aus Vinylacetat und Essigsäure über Kopf abgetrennt, welches in einem weiteren Destillationsschritt aufgetrennt wurde. Das Sumpfprodukt der ersten Destillation wurde einer weiteren Destillation unterzogen. Das Zielprodukt Vinyllaurat konnte daraus als Kopfprodukt mit einer Reinheit von > 99,0 Gew.-% und ohne Polymerbestandteile produziert werden.

### Beispiel 2:

In einem kontinuierlich betriebenen Rohrreaktor wurden 14 kg/h Vinylacetat, 6 kg/h Laurinsäure, 30 kg/h Rücklaurinsäure, welche neben Ru-Katalysator, polymeren Nebenprodukten und Vinyllaurat noch ein Gemisch aus Laurinsäure und Laurinsäureanhydrid enthielt, und 12 kg/h der nach der Entspannung des Reaktionsgemisches erhaltenen Gasphase als Kondensat in den Reaktor dosiert. Das molare Verhältnis zwischen Vinylacetat und Laurinsäure betrug 4 : 1.

Die Reaktion wurde bei 140°C und 7 bar abs. durchgeführt. Es wurde kontinuierlich Reaktionsgemisch entnommen und auf Atmosphärendruck entspannt. Dabei fielen etwa 20 Gew.-% des entnommenen Reaktionsgemisches als Dampfphase an mit ca. 96 Gew.-% Vinylacetat und ca. 4 Gew.-% Essigsäure, sowie Spuren von Vinyllaurat und Laurinsäure. Diese Dampfphase wurde abgetrennt, komprimiert und als Kondensat in den Reaktor zurückgeführt.

Bei einer Betriebszeit von ca. 1000 Stunden schwankte die Ausbeute in Abhängigkeit vom Polymer- und Katalysatorgehalt zwischen 60 % und 75 %

Aus der nach Entspannung erhaltenen flüssigen Phase wurde in der ersten Destillation ein Gemisch aus Vinylacetat und Essigsäure über Kopf abgetrennt, welches in einem weiteren Destillationsschritt aufgetrennt wurde. Das Sumpfprodukt der ersten Destillation wurde einer weiteren Destillation unterzogen. Das Zielprodukt Vinyllaurat konnte daraus als Kopfprodukt mit einer Reinheit von > 99,0 Gew.-% und ohne Polymerbestandteile produziert werden.

### Vergleichsbeispiel 3:

In einem kontinuierlich betriebenen Rohrreaktor wurden 30 kg/h Vinylacetat, 7 kg/h Neodecansäure und 23 kg/h Rückneodecansäure, welche neben Ru-Katalysator und polymeren Nebenprodukten und Neodecansäurevinylester noch Neodecansäure enthielt, in den Reaktor dosiert. Auf die Rückführung der durch Entspannung generierten Gasphase wurde hier verzichtet. Das molare Verhältnis zwischen Vinylacetat und Neodecansäure betrug 4 : 1.

Die Reaktion wurde bei 140°C und 7 bar abs. durchgeführt. Es wurde kontinuierlich Reaktionsgemisch entnommen und auf Atmosphärendruck entspannt. Dabei fielen etwa 20 Gew.-% des entnommenen Reaktionsgemisches als Dampfphase an, welche nicht zurückgeführt wurde. Die Dampfphase enthielt 92 Gew.-% Vinylacetat, 6 Gew.-% Essigsäure, 1,5 Gew.-% Neodecansäurevinylester und 0,5 Gew.-% Neodecansäure.

Bei einer Betriebszeit von ca. 1000 Stunden betrug die Ausbeute in Abhängigkeit vom Polymer- und Katalysatorgehalt zwischen 45 % und 75 %.

Aus der nach Entspannung erhaltenen flüssigen Phase wurde in der ersten Destillation ein Gemisch aus Vinylacetat und Essigsäure über Kopf abgetrennt, welches in einem weiteren Destillationsschritt aufgetrennt wurde. Das Sumpfprodukt der ersten Destillation wurde einer weiteren Destillation unterzogen. Das Zielprodukt Neodecansäurevinylester konnte als Kopfprodukt mit einer Reinheit von > 99,0 Gew.-% und ohne Polymerbestandteile produziert werden.

### Beispiel 4:

In einem kontinuierlich betriebenen Rohrreaktor wurden 19 kg/h Vinylacetat, 7 kg/h Neodecansäure, 23 kg/h Rückneodecansäure, welche neben Ru-Katalysator noch polymere Nebenprodukte und Neodecansäurevinylester und Neodecansäure enthielt und 12 kg/h der nach Entspannung erhaltenen Dampfphase (in kondensierter Form) in den Reaktor dosiert. Das molare Verhältnis zwischen Vinylacetat und Neodecansäure betrug ebenfalls 4 : 1.

Die Reaktion wurde bei 140°C und 7 bar abs. durchgeführt. Es wurde kontinuierlich Reaktionsgemisch entnommen und auf Atmosphärendruck entspannt. Dabei fielen etwa 20 Gew.-% des entnommenen Reaktionsgemisches als Dampfphase an mit 92 Gew.-% Vinylacetat und 6 % Essigsäure, 1,5 % Neodecansäurevinylester und 0,5 % Neodecansäure. Diese Dampfphase wurde abgetrennt, gekühlt und als Kondensat in den Reaktor zurückgeführt.

Bei einer Betriebszeit von 1000 Stunden betrug die Ausbeute in Abhängigkeit vom Polymer- und Katalysatorgehalt zwischen 60 % und 75 %

Aus der nach Entspannung erhaltenen flüssigen Phase wurde in der ersten Destillation ein Gemisch aus Vinylacetat und Essigsäure über Kopf abgetrennt, welches in einem weiteren Destillationsschritt aufgetrennt wurde. Das Sumpfprodukt der ersten Destillation wurde einer weiteren Destillation unterzogen. Das Zielprodukt Neodecansäurevinylester konnte als Kopfproduktmit einer Reinheit von > 99.0 Gew.-% und ohne Polymerbestandteile produziert werden.

Der Vergleich von Beispiel 2 mit Vergleichsbeispiel 1 oder von Beispiel 4 mit Vergleichsbeispiel 3 zeigt, dass mit dem erfindungsgemäßen Verfahren die Rückführung der bei der Entspannung erhaltenen Gasphase möglich ist, ohne dass dadurch die Qualität oder die Ausbeute abnimmt. Die mit der Rückführung der Gasphase resultierenden Einsparungen können ohne Minderung von Qualität und Ausbeute realisiert werden.

## Patentansprüche

1. Verfahren zur Umvinylierung einer Eduktcarbonsäure mit einem Eduktvinylester zu einem Produktvinylester und der korrespondierenden Säure des Eduktvinylesters in Gegenwart von einem oder mehreren Katalysatoren, in einem Reaktor bei einer Temperatur von 100°C bis 170°C und einem Druck von 2 bar abs. bis 15 bar abs., wobei der Eduktvinylester, die Eduktcarbonsäure und der Katalysator einem Reaktor zugeführt werden, und die Umvinylierungsreaktion durchgeführt wird, und nach Abschluss der Umvinylierungsreaktion der Produktvinylester vom Reaktionsgemisch destillativ abgetrennt wird, **dadurch gekennzeichnet, dass** das Reaktionsgemisch in einem Entspannungsbehälter entspannt wird, die dabei anfallende gasförmige Phase anschließend kondensiert wird und ohne weitere Aufreinigung in den Reaktor zurückgeführt wird, und die dabei anfallende flüssige Phase in einer ersten Destillation von Eduktvinylester und korrespondierender Säure befreit wird, und in einem weiteren Destillationsschritt der Produktvinylester von dem Reaktionsgemisch abgetrennt wird.

2. Verfahren nach Anspruch 1, **dadurch gekennzeichnet, dass** in dem weiteren Destillationsschritt der Produktvinylester als Kopfprodukt von den restlichen Bestandteilen abgetrennt wird, falls der Siedepunkt des Produktvinylesters niedriger ist als der Siedepunkt der Eduktcarbonsäure.

3. Verfahren nach Anspruch 1, **dadurch gekennzeichnet, dass** in dem weiteren Destillationsschritt, falls der Siedepunkt des Produktvinylesters höher ist als der Siedepunkt der Eduktcarbonsäure, zunächst ein Gemisch von Produktvinylester und Eduktcarbonsäure als Kopfprodukt vom katalysatorhaltigen Rest abgetrennt wird, und dieses Gemisch dann in einer weiteren Destillationsvorrichtung zur Herstellung des -reinen Produktvinylesters aufgetrennt wird.

4. Verfahren nach Anspruch 1 bis 3, **dadurch gekennzeichnet, dass** als Eduktvinylester ein Carbonsäurevinylester der allgemeinen Formel R-C(O)-O-CH=CH₂ eingesetzt wird, wobei R ein aliphatischen Rest mit 1 bis 12 C-Atomen ist, oder ein cycloaliphatischer Rest mit bis zu 12 C-Atomen ist, oder ein aromatischer Rest mit bis zu 12 C-Atomen ist.

5. Verfahren nach Anspruch 1 bis 3, **dadurch gekennzeichnet, dass** als Eduktvinylester Vinylacetat eingesetzt wird.

6. Verfahren nach Anspruch 1 bis 5, **dadurch gekennzeichnet, dass** als Eduktcarbonsäure eine Carbonsäure der allgemeinen Formel R'-COOH eingesetzt wird, wobei R' ein aliphatischer Rest mit 1 bis 22 C-Atomen ist, oder ein cycloaliphatischer Rest mit bis zu 22 C-Atomen ist, oder ein aromatischer Rest mit bis zu 22 C-Atomen ist.

7. Verfahren nach Anspruch 1 bis 5, **dadurch gekennzeichnet, dass** als Eduktcarbonsäure eine Carbonsäure eingesetzt wird aus der Gruppe umfassend Versaticsäuren und Neo-Säuren, jeweils mit 9 bis 12 C-Atomen, und Fettsäuren wie Laurinsäure, Myristinsäure, Palmitinsäure, Stearinsäure.

8. Verfahren nach Anspruch 1, 2 und 4 bis 7, **dadurch gekennzeichnet, dass** als Eduktcarbonsäure eine Carbonsäure eingesetzt wird, deren Siedepunkt höher ist, als der Siedepunkt des Produktvinylesters.

## Claims

1. Process for transvinylation of a reactant carboxylic acid with a reactant vinyl ester to give a product vinyl ester and the corresponding acid of the reactant vinyl ester in the presence of one or more catalysts, in a reactor at a temperature of 100°C to 170°C and a pressure of 2 bar abs. to 15 bar abs., wherein the reactant vinyl ester, the reactant carboxylic acid and the catalyst are supplied to a reactor and the transvinylation reaction is conducted and, on completion of the transvinylation reaction, the product vinyl ester is separated from the reaction mixture by distillation, **characterized in that** the reaction mixture is decompressed in a decompression vessel, the gaseous phase obtained is then condensed and recycled into the reactor without further purification, and the liquid phase obtained is freed of reactant vinyl ester and corresponding acid in a first distillation, and the product vinyl ester is separated from the reaction mixture in a further distillation step.

2. Process according to Claim 1, **characterized in that**, in the further distillation step, the product vinyl ester is separated as top product from the residual constituents if the boiling point of the product vinyl ester is lower than the boiling point of the reactant carboxylic acid.

3. Process according to Claim 1, **characterized in that**, in the further distillation step, if the boiling point of the product vinyl ester is higher than the boiling point of the reactant carboxylic acid, initially a mixture of product vinyl ester and reactant carboxylic acid is removed as top product from the catalyst-containing residue and this mixture is then separated in a further distillation apparatus for producing the pure product vinyl ester.

4. Process according to Claim 1 to 3, **characterized in that** a carboxylic vinyl ester of the general formula R-C(O)-O-CH=CH₂ is used as reactant vinyl ester, where R is an aliphatic residue having 1 to 12 carbon atoms, or is a cycloaliphatic residue having up to 12 carbon atoms, or is an aromatic residue having up to 12 carbon atoms.

5. Process according to Claim 1 to 3, **characterized in that** vinyl acetate is used as reactant vinyl ester.

6. Process according to Claim 1 to 5, **characterized in that** a carboxylic acid of the general formula R'-COOH is used as reactant carboxylic acid, where R' is an aliphatic residue having 1 to 22 carbon atoms, or is a cycloaliphatic residue having up to 22 carbon atoms, or is an aromatic residue having up to 22 carbon atoms.

7. Process according to Claim 1 to 5, **characterized in that** the reactant carboxylic acid used is a carboxylic acid from the group comprising versatic acids and neo acids, in each case having 9 to 12 carbon atoms, and fatty acids such as lauric acid, myristic acid, palmitic acid and stearic acid.

8. Process according to Claim 1, 2 and 4 to 7, **characterized in that** the reactant carboxylic acid used is a carboxylic acid whose boiling point is higher than the boiling point of the product vinyl ester.

## Revendications

1. Procédé de transvinylation d'un acide carboxylique réactif avec un ester de vinyle réactif pour former un ester de vinyle produit et l'acide correspondant de l'ester de vinyle réactif en présence d'un ou de plusieurs catalyseurs, dans un réacteur à une température de 100 °C à 170 °C et à une pression de 2 bar abs. à 15 bar abs., l'ester de vinyle réactif, l'acide carboxylique réactif et le catalyseur étant introduits dans un réacteur, et la réaction de transvinylation étant réalisée et, après la fin de la réaction de transvinylation, l'ester de vinyle produit étant séparé par distillation du mélange réactionnel, **caractérisé en ce que** le mélange réactionnel est détendu dans un contenant de détente, puis la phase gazeuse formée est condensée et recyclée dans le réacteur sans purification supplémentaire, et la phase liquide formée est débarrassée lors d'une première distillation de l'ester de vinyle réactif et de l'acide correspondant, et, lors d'une étape de distillation supplémentaire, l'ester de vinyle produit est séparé du mélange réactionnel.

2. Procédé selon la revendication 1, **caractérisé en ce que**, lors de l'étape de distillation supplémentaire, l'ester de vinyle produit est séparé des constituants restants sous la forme d'un produit de tête si le point d'ébullition de l'ester de vinyle produit est inférieur au point d'ébullition de l'acide carboxylique réactif.

3. Procédé selon la revendication 1, **caractérisé en ce que**, lors de l'étape de distillation supplémentaire, si le point d'ébullition de l'ester de vinyle produit est supérieur au point d'ébullition de l'acide carboxylique réactif, tout d'abord un mélange de l'ester de vinyle produit et de l'acide carboxylique réactif est séparé du résidu contenant le catalyseur sous la forme d'un produit de tête, puis ce mélange est séparé dans un dispositif de distillation supplémentaire pour la fabrication de l'ester de vinyle produit pur.

4. Procédé selon les revendications 1 à 3, **caractérisé en ce qu'**en tant qu'ester de vinyle réactif, un ester de vinyle d'acide carboxylique de formule générale R-C(O)-O-CH=CH₂ est utilisé, R étant un radical aliphatique de 1 à 12 atomes C, ou un radical cycloaliphatique contenant jusqu'à 12 atomes C, ou un radical aromatique contenant jusqu'à 12 atomes C.

5. Procédé selon les revendications 1 à 3, **caractérisé en ce que** de l'acétate de vinyle est utilisé en tant qu'ester de vinyle réactif.

6. Procédé selon les revendications 1 à 5, **caractérisé en ce qu'**en tant qu'acide carboxylique réactif, un acide carboxylique de formule générale R'-COOH est utilisé, R' étant un radical aliphatique de 1 à 22 atomes C, ou un radical cycloaliphatique contenant jusqu'à 22 atomes C, ou un radical aromatique contenant jusqu'à 22 atomes C.

7. Procédé selon les revendications 1 à 5, **caractérisé en ce qu'**en tant qu'acide carboxylique réactif, un acide carboxylique du groupe comprenant les acides versatiques et les néo-acides, contenant chacun 9 à 12 atomes C, et les acides gras tels que l'acide laurique, l'acide myristique, l'acide palmitique, l'acide stéarique, est utilisé.

8. Procédé selon les revendications 1, 2 et 4 à 7, **caractérisé en ce qu'**en tant qu'acide carboxylique réactif, un acide carboxylique dont le point d'ébullition est supérieur au point d'ébullition de l'ester de vinyle produit est utilisé.
